Europäisches Patentamt

(19) European Patent Office

Office européen des brevets

(11) Publication number: **0 021 511**
**B1**

(12) **EUROPEAN PATENT SPECIFICATION**

(45) Date of publication of patent specification: **25.05.83**

(51) Int. Cl.³: **C 07 C 39/08, C 07 C 37/00**

(21) Application number: **80200552.0**

(22) Date of filing: **13.06.80**

(54) Process for the preparation of optionally alkyl-substituted resorcinol and catalyst to be used in this process.

(30) Priority: **28.06.79 NL 7905024**

(43) Date of publication of application:
**07.01.81 Bulletin 81/1**

(45) Publication of the grant of the patent:
**25.05.83 Bulletin 83/21**

(84) Designated Contracting States:
**BE CH DE FR GB IT LI NL**

(56) References cited:
**EP - A - 0 008 322**
**FR - A - 2 288 724**
**US - A - 3 402 210**
**US - A - 3 950 438**

**CHEMICAL ABSTRACTS, vol. 77, no. 10,**
**September 4, 1972, page 330, abstract 66682v**
**COLUMBUS, Ohio (US)**
**CHEMICAL ABSTRACTS, vol. 89, no. 18,**
**October 30, 1978, page 413, abstract 153329b**
**COLUMBUS, Ohio (US) L. S. KRAVCHUK et al.:**
**"Study of palladium-alumina catalysts**
**containing cerium (IV)"**

(73) Proprietor: **STAMICARBON B.V.**
**Postbus 10**
**NL-6160 MC Geleen (NL)**

(72) Inventor: **Van de Moesdijk, Cornelis Gerardus**
**Maria**
**Drossaert Saldenstraat 7**
**NL-6181 ER Elsloo (NL)**
Inventor: **Kleuskens, Engelina Catherina**
**Burg. van Mulkenstraat 4**
**NL-6181 CV Elsloo (NL)**
Inventor: **Janssen, Petrus Hubertus Joseph**
**Mauritspark 32**
**NL-6143 HN Geleen (NL)**
Inventor: **Boelens, Christiaan**
**Pater Pelzersstraat 48**
**NL-6125 CB Obbicht (NL)**

(74) Representative: **Pinckaers, August René et al,**
**OCTROOIBUREAU DSM Postbus 9**
**NL-6160 MA Geleen (NL)**

Courier Press, Leamington Spa, England.

(56) References cited:

**CHEMICAL ABSTRACTS, vol. 86, no. 17, April 25, 1977, page 504 abstract 120837a COLUMBUS, Ohio (US) N. R. BURSIAN et al.: "Effect of a series of periodic system elements on the catalytic and structural properties of platinum on silica gel"**

# 0 021 511

Process for the preparation of optionally alkyl-substituted resorcinol and
catalyst to be used in this process

The invention relates to a process for the preparation of optionally alkyl-substituted resorcinol by conversion of an ester of optionally alkyl-substituted 4-oxopentane-1-carboxylic acid in the gaseous state using a dehydrogenation catalyst, and to the catalyst to be used in this process.

This process has been described in United States Patent Specification 3,950,438. On further experimental examination of the methodology described in said patent specification it has been found that when applying known dehydrogenation catalysts, such as, for instance, platinum on carbon, the conversion of the ester in question and the resorcinol efficiency decrease fairly rapidly, for instance after an operating time of 40 hours, to a value that is unacceptable for practical purposes.

Other known dehydrogenation catalysts are disclosed in FR—A—2 288 724, US—A—3 420 210, Chemical Abstracts vol. 77, no. 10, September 4, 1972 page 330, abstract 66682v, Chemical Abstracts vol. 89, no. 18, October 30, 1978, page 413, abstract 153329b and Chemical Abstracts vol. 86, no. 17, April 25, 1977, page 504 abstract 120837a.

The non-prepublished EP—A—0 008 322 discloses a catalyst for the manufacture of resorcinols which comprises two components e.g. Pt on charcoal and Th on charcoal whereby it is essential that the catalyst-components are prepared separately and then brought together.

The present invention provides a dehydrogenation catalyst allowing the efficiency and the conversion to be maintained for a long time at a value that is more suitable for practical purposes.

The process according to the invention for the preparation of optionally alkyl-substituted resorcinol by conversion of an ester of optionally alkyl-substituted 4-oxopentane-1-carboxylic acid in the gaseous state using a dehydrogenation catalyst is characterized in that one or more metals selected from the group consisting of platinum, palladium, ruthenium, iridium and rhodium on a carrier is used as dehydrogenation catalyst, with as carrier a combination of one or more oxides selected from the group consisting of silicon dioxide, aluminium oxide and titanium oxide and one or more oxides selected from the group consisting of thorium oxide and lanthanide oxides, whereby the dehydrogenation catalyst is prepared by precipitating the catalyst on the combination of oxides. The process according to the invention can be carried out starting from various esters of the 4-oxopentane-1-carboxylic acid such as, for instance, alkyl-, cycloalkyl-, and phenyl-esters. Very suitable are alkyl-esters with an alkyl group containing up to 6 carbon atoms. In the process according to the invention a univalent alcohol is formed from the ester group, for instance methyl alcohol when methyl ester of said carboxylic acid is started from. The alcohol formed can be recovered from the reaction mixture and be used for the preparation of the starting product. Optionally, the starting product can be substituted by an alkyl group in the 1st, 2nd, 3rd and/or 5th positions. The total number of carbon atoms of these substituents will preferably not be chosen higher than 12, and the number of carbon atoms of one substituent preferably not higher than 6.

The noble metal content of the catalyst according to the invention can be varied, for instance between 0.1 and 15% by weight of noble metal. By preference a content of 0.5—5% by weight of noble metal is used. As noble metal platinum is particularly suitable.

The catalyst according to the invention can be used on a carrier chosen from various combinations of one or more oxides selected from the group consisting of silicon dioxide, aluminium oxide and titanium oxide and the other group of oxides in question. By preference the combination of silicon dioxide and thorium oxide is applied as carrier in a relative weight ratio of 1—80 grams of thorium oxide, in particular 5—15 grams of thorium oxide per 100 grams of silicon dioxide. The porosity of the silicon dioxide may vary. A good result can be obtained with silicon dioxide of medium porosity, i.e. silicon dioxide the average pore radius of which is between 2 and 5 nanometers (measured by capillary condensation of nitrogen for the 1—30 nanometer range).

The process according to the invention can be carried out in various ways. The gaseous starting product can be diluted with an inert gas such as, for instance, nitrogen and carbon dioxide and be contacted with the catalyst in the form of a fixed bed or a fluid bed. The gaseous starting product can also be contacted with the catalyst in the presence of hydrogen, which has a favourable influence upon the activity of the catalyst. The space velocity may be varied, for instance between 0.05 and 5 grams of ester per milliliter of catalyst per hour. Usually a space velocity of between 0.1 and 1.5 grams of ester per milliliter of catalyst is applied.

The process according to the invention may be carried out at various reaction temperatures. By preference a temperature between 200 and 400°C is applied. The gaseous reaction mixture obtained at such temperatures can be cooled, with the possibility of utilizing the heat to be discharged, to form a condensate, after which the desired product can be recovered from this condensate by fractionated distillation or extraction.

Besides the desired product, the reaction mixture obtained contains non-converted ester, which can be recycled, and possibly also a small amount of the 4-oxopentane-1-carboxylic acid corresponding to the ester and/or the lactone of the acid in question. This small amount of the 4-oxopentane-1-carboxylic acid and/or the lactone can be separated off and be converted into the ester in question. It has, however, been found that recycling of the acid and/or lactone in question together with the non-

3

converted ester in an amount of at most 0.4 moles of acid and/or lacton per mole of the ester to be passed over the catalyst does not have any adverse consequences.

In the following examples the invention will be further elucidated.

Catalyst preparation

Silicon dioxide lumps (49.6 grams, diameter 3—8 mm, average pore radius 3.6 nm, type silicon dioxide gel M of the firm Hermann at Cologne, W. Germany) are, to avoid fracturing, immersed in a beaker filled with approximately 90 ml of acetone. Subsequently 100 ml of water is added whilst stirring, after which the lumps are filtered off and washed three times with water.

The silicon dioxide thus moistened is then mixed with a solution of 11.8 grams of $Th(NO_3)_4 \cdot 4 H_2O$ in 60 ml of water in an evaporation dish. The mixture is evaporated on a water bath whilst being stirred and subsequently dried for 1 hour at 110°C. The dried product is then calcined for 2 hours at 550°C.

The carrier obtained is subsequently mixed with a solution of 1.32 grams of $H_2PtCl_6 \cdot 6 H_2O$ in 50 ml of water, the mixture obtained is evaporated whilst being stirred and the evaporated product is dried for 16 hours at 110°C.

The dried product is finally introduced into a reactor (as described hereafter in the examples) and activated for 16 hours at 350°C by passing hydrogen over it.

The yield is 56 grams of catalyst containing 1% by weight of platinum and 11.4% by weight of $ThO_2$ (10% by weight of Th).

In a similar way other catalysts according to the invention can be prepared. The metal oxide required for the carrier can be obtained from various salts, for instance $CeO_2$ from $Ce(NO_3)_2 \cdot 6 H_2O$ or $Nd_2O_3$ from $NdCl_3$.

Example 1

Through a vertical tubular reactor (18 mm in diameter and 400 mm in length), in which there is a zone of 25 ml (bulk volume) of catalyst, a gaseous mixture of nitrogen, hydrogen and methyl ester of 4-oxopentane-1-carboxylic acid is made to descend for about 845 hours. The catalyst zone is on both sides bounded by a zone of 25 ml of inert ceramic material. As catalyst, platinum on a carrier of silicon dioxide and thorium oxide (prepared and activated in the way indicated above, 1% by weight of Pt, 10% by weight of Th) is applied.

The gaseous mixture (4 moles of hydrogen and 2 moles of nitrogen per mole of methyl ester) is obtained by evaporating liquid methyl ester and mixing the vapour with hydrogen and nitrogen. Per milliliter (bulk volume) of catalyst 0.2 grams/hour of methyl ester are passed through. The first 400 hours the temperature of the catalyst is kept at 320°C, by means of a heating jacket around the reactor, and afterwards at 350°C.

The composition of the reaction mixture obtained is determined a number of times by passing the gas mixture obtained for some time, for instance 1 hour, through two coupled vessels, which are cooled to 0°C and —80°C respectively, and analysing the condensed product thus obtained gas chromatographically.

The weight and the composition of the condensed product obtained are given in the table below. This table also indicates the calculated conversion of the ester and the efficiencies of the products obtained as well as the operating time that has passed as soon as the gaseous reaction mixture is led through the cooled vessels.

| Operating time in hours | Weight of product in grams | Composition (% by weight) | | | | Efficiency (mol %) | | | Conversion % |
|---|---|---|---|---|---|---|---|---|---|
| | | Resorcinol | Dihydro-resorcinol | Phenol | methyl ester | Resorcinol | Dihydro-resorcinol | Phenol | |
| 43 | 16.2 | 24.6 | 4.2 | 5.4 | 34.9 | 36.4 | 6.2 | 9.4 | 71.7 |
| 89 | 8.1 | 20.5 | 4.6 | 3.9 | 46.4 | 38.7 | 8.5 | 8.6 | 59.9 |
| 185.5 | 27.8 | 16.3 | 4.5 | 2.6 | 56.2 | 36.9 | 9.9 | 6.9 | 50.7 |
| 235.5 | 4.0 | 17.7 | 4.9 | 2.8 | 58.9 | 40.1 | 10.9 | 7.4 | 49.5 |
| 334.5 | 9.2 | 14.5 | 4.2 | 2.2 | 60.8 | 35.1 | 10.4 | 6.2 | 29.8 |
| 402 | 5.6 | 33.1 | 4.6 | 4.3 | 40.0 | 50.3 | 6.9 | 7.7 | 68.3 |
| 500 | 5.0 | 26.0 | 5.4 | 3.0 | 45.1 | 45.9 | 9.4 | 6.2 | 60.8 |
| 596 | 9.6 | 24.1 | 5.6 | 2.8 | 47.1 | 48.1 | 10.9 | 6.5 | 58.3 |
| 741 | 4.5 | 20.4 | 5.6 | 2.0 | 54.5 | 46.3 | 12.6 | 5.2 | 51.4 |
| 859 | 6.3 | 20.4 | 5.4 | 3.2 | 49.3 | 41.7 | 10.9 | 7.6 | 56.5 |

Comparative Example 1

The experiment described in Example 1 is repeated for about 115 hours at a catalyst temperature of 320°C with the same type of catalyst, but without $ThO_2$ (1% by weight of Pt on silicon dioxide). After an operating time of 42 hours, the conversion of the methyl ester amounts to 27.6%. Of the converted methyl ester 18.8% has been converted into resorcinol, 8% into dihydroresorcinol and 13% into phenol.

After an operating time of 114 hours the conversion of the methyl ester amounts to 17.3%. Of the amount of methyl ester converted then 13% has been converted into resorcinol, 17.7% into dihydroresorcinol and 18.5% into phenol.

Comparative Example 2

The experiment described in Example 1 is repeated for about 42 hours at a catalyst temperature of 350°C with the same type of catalyst, but with carbon instead of silicon dioxide. After an operating time of 41 hours the conversion of the methyl ester amounts to 33%. Of the methyl ester converted 4.9% has been converted into resorcinol, 39.6% into dihydroresorcinol and 3.8% into phenol. During this experiment the weight of the catalyst appears to have increased by approximately 27% as a result of tar deposition.

Example II

The experiment described in Example I is repeated for about 740 hours at a catalyst temperature of 350°C. In doing so, the amounts of hydrogen and nitrogen (expressed in moles per mole of ester) in the gaseous starting compound as well as the space velocity (expressed in grams of ester per ml of catalyst per hour) are varied some hours before the analytical measurements.

The pertaining data and results have been tabulated below.

| Operating time in hours | Space velocity | $N_2$ | $H_2$ | Weight of product in grams | Composition (% by weight) | | | | Efficiency (mole %) | | | Conversion % |
|---|---|---|---|---|---|---|---|---|---|---|---|---|
| | | | | | Resorcinol | Dihydro-resorcinol | Phenol | Methyl ester | Resorcinol | Dihydro-resorcinol | Phenol | |
| 41 | 0.17 | 4.0 | 8.0 | 7.2 | 24.3 | 2.0 | 31.9 | 9.5 | 22.7 | 1.8 | 35.0 | 93.6 |
| 66 | 0.17 | 4.0 | 8.0 | 7.8 | 14.8 | 1.9 | 30.3 | 15.9 | 18.3 | 2.4 | 44.0 | 86.9 |
| 89.5 | 0.17 | 8.0 | 16.0 | 8.6 | 27.7 | 3.7 | 18.5 | 15.4 | 27.3 | 3.6 | 21.3 | 89.7 |
| 256.5 | 0.17 | 8.0 | 16.0 | 6.0 | 41.4 | 8.2 | 8.9 | 27.3 | 38.9 | 7.6 | 9.8 | 83.6 |
| 353.5 | 0.17 | 8.0 | 16.0 | 4.0 | 39.5 | 8.5 | 8.4 | 27.4 | 31.6 | 6.7 | 7.0 | 85.6 |
| 378 | 0.5 | 4.0 | 8.0 | 20.4 | 19.5 | 8.8 | 2.5 | 56.3 | 45.6 | 20.2 | 6.9 | 49.9 |
| 425.5 | 0.5 | 4.0 | 8.0 | 31.4 | 16.8 | 8.0 | 1.9 | 62.0 | 45.1 | 21.1 | 6.1 | 44.0 |
| 569 | 0.5 | 4.0 | 8.0 | 20.2 | 14.2 | 8.5 | 1.5 | 61.3 | 39.0 | 23.0 | 4.7 | 43.7 |
| 593 | 1.0 | 2.0 | 4.0 | 25.0 | 8.4 | 7.6 | 0.7 | 72.5 | 35.5 | 31.6 | 3.5 | 29.9 |
| 665.5 | 1.0 | 2.0 | 4.0 | 68.9 | 7.3 | 7.5 | 0.6 | 74.8 | 32.2 | 32.5 | 2.9 | 28.5 |
| 689 | 1.0 | 3.0 | 6.0 | 28.1 | 6.8 | 7.3 | 0.6 | 75.6 | 30.4 | 32.0 | 2.9 | 28.1 |
| 713 | 1.0 | 3.0 | 6.0 | 68.4 | 6.5 | 7.5 | 0.6 | 76.4 | 24.4 | 27.7 | 2.4 | 31.2 |
| 738 | 1.0 | 1.0 | 2.0 | 20.0 | 6.3 | 7.5 | 0.5 | 75.2 | 25.1 | 25.7 | 2.3 | 30.2 |

# 0 021 511

Example III

In the way described in Example I the isopropyl ester of 4-oxopentane-1-carboxylic acid is for approximately 75 hours converted at a catalyst temperature of 350°C, using the same type of catalyst as in Example I. Per mole of isopropyl ester 8 moles of hydrogen and 4 moles of nitrogen are used. The space velocity is 0.8 grams of ester per milliliter of catalyst per hour.

After an operating time of 72 hours the conversion of the ester amounts to 45.3%. Of the amount of ester converted 33.1% has been converted into resorcinol, 27.7% into dihydroresorcinol and 5.7% into phenol.

Example IV

In the way described in Example I the ethyl ester of 4-oxopentane-1-carboxylic acid is converted for about 760 hours at a catalyst temperature of 320°C, using the same type of catalyst as in Example I.

The space velocity is 0.2 grams of ethyl ester per milliliter of catalyst per hour. After an operating time of 480 hours, for 50 hours 6 moles of $H_2$ per mole of ester are applied instead of 2 moles of $N_2$ and 4 moles of $H_2$ per mole of ester.

The pertaining data and results have been tabulated below.

8

| Operating time in hours | Weight of product in grams | Composition (% by weight) | | | | Efficiency (mole %) | | | Conversion % |
|---|---|---|---|---|---|---|---|---|---|
| | | Resorcinol | Dihydro-resorcinol | Phenol | Methyl ester | Resorcinol | Dihydro-resorcinol | Phenol | |
| 40 | 3.74 | 22.8 | 1.7 | 8.0 | 25.4 | 30.3 | 2.2 | 12.5 | 81.0 |
| 138 | 8.23 | 27.6 | 3.1 | 6.9 | 39.6 | 45.6 | 4.9 | 13.4 | 68.7 |
| 185.5 | 4.8 | 20.2 | 2.7 | 5.4 | 50.1 | 54.4 | 7.2 | 17.1 | 51.6 |
| 233.5 | 38.2 | 19.6 | 3.1 | 5.7 | 46.3 | 38.6 | 6.0 | 13.2 | 61.2 |
| 353.5 | 24.0 | 18.9 | 3.7 | 5.1 | 51.5 | 38.8 | 7.5 | 12.2 | 57.5 |
| 406.5 | 4.6 | 18.2 | 3.0 | 5.4 | 52.4 | 46.8 | 7.6 | 16.1 | 57.6 |
| 476 | 13.6 | 16.1 | 3.5 | 4.1 | 54.4 | 37.6 | 8.0 | 11.3 | 53.0 |
| 504 | 3.2 | 15.8 | 3.1 | 6.7 | 49.9 | 26.3 | 5.1 | 13.1 | 63.4 |
| 522 | 11.8 | 14.5 | 2.9 | 6.7 | 50.4 | 31.2 | 6.1 | 16.7 | 57.0 |
| 547 | 4.5 | 17.0 | 3.3 | 4.6 | 55.2 | 39.6 | 7.6 | 12.6 | 52.8 |
| 613 | 4.4 | 16.1 | 3.2 | 4.4 | 56.4 | 38.0 | 7.4 | 12.0 | 51.9 |
| 636.5 | 4.2 | 14.9 | 3.5 | 4.1 | 59.0 | 43.2 | 10.1 | 13.8 | 44.0 |
| 756 | 4.5 | 13.1 | 3.1 | 3.1 | 62.2 | 35.9 | 8.4 | 9.9 | 45.7 |

Example V

The experiment described in Example I is repeated for about 385 hours using as catalyst platinum on a carrier of silicon dioxide with $CeO_2$ (1% by weight of Pt and 5% by weight of Ce).

The space velocity is 0.2 grams of ester per milliliter of catalyst per hour. The first 190 hours 6.2 moles of $N_2$ and 14.3 moles of $H_2$ are used per mole of ester, and afterwards 3.9 moles of $N_2$ and 7.8 moles $H_2$.

The first 335 hours the catalyst is kept at 350°C and afterwards at 320°C.

In the table below the results are indicated.

| Operating time in hours | Weight of product in grams | Composition (% by weight) | | | | Efficiency (mole %) | | | Conversion in % |
|---|---|---|---|---|---|---|---|---|---|
| | | Resorcinol | Dihydro-resorcinol | Phenol | Methyl ester | Resorcinol | Dihydro-resorcinol | Phenol | |
| 41 | 2.6 | 21.6 | 1.5 | 22.7 | 12.0 | 18.7 | 1.3 | 22.9 | 92.7 |
| 115.5 | 4.1 | 25.4 | 3.4 | 13.7 | 30.1 | 33.8 | 4.4 | 21.3 | 76.6 |
| 161.5 | 4.5 | 24.9 | 3.9 | 9.4 | 33.0 | 39.4 | 6.0 | 17.5 | 71.5 |
| 187.5 | 4.3 | 21.2 | 3.0 | 13.1 | 24.5 | 31.5 | 4.4 | 22.9 | 76.9 |
| 210.5 | 3.7 | 27.3 | 3.5 | 11.7 | 31.4 | 36.8 | 4.6 | 18.5 | 75.5 |
| 311 | 4.9 | 27.4 | 3.5 | 12.0 | 26.3 | 33.1 | 4.2 | 16.9 | 80.5 |
| 311.5 | 5.6 | 28.0 | 4.2 | 9.1 | 31.2 | 41.4 | 6.0 | 15.8 | 73.9 |
| 353 | 8.5 | 13.4 | 3.9 | 4.1 | 57.1 | 29.0 | 8.3 | 10.4 | 51.5 |
| 383 | 4.7 | 13.6 | 4.0 | 4.2 | 57.6 | 39.9 | 11.5 | 14.4 | 45.3 |

# 0 021 511

## Claims

1. Process for the preparation of optionally alkyl-substituted resorcinol by conversion of an ester of optionally alkyl-substituted 4-oxopentane-1-carboxylic acid in the gaseous state using a dehydrogenation catalyst, this process being characterized in that one or more metals selected from the group consisting of platinum, palladium, ruthenium, iridium, and rhodium on a carrier are used as dehydrogenation catalyst, with as carrier a combination of one or more oxides selected from the group consisting of silicon dioxide, aluminium oxide and titanium oxide and one or more oxides selected from the group consisting of thorium oxide and lanthanide oxides, whereby the dehydrogenation catalyst is prepared by precipitating the catalyst on the combination of oxides.

2. Process according to claim 1, characterized in that a dehydrogenation catalyst is used with a content of 0.5—5% by weight of metal of said group of metals.

3. Process according to either claim 1 or 2, characterized in that platinum is used as metal.

4. Process according to any of the claims 1—3, characterized in that the combination silicon dioxide thorium oxide is used as carrier.

5. Process according to claim 4, characterized in that silicon dioxide having an average pore radius between 2 and 5 nanometers is used.

6. Process according to either claim 4 or 5, characterized in that 5—15 grams of thorium oxide are used per 100 grams of silicon dioxide.

7. Process according to any of the claims 1—6, characterized in that the acid and/or lactone formed during the conversion of the ester is recycled and per mole of ester to be passed over the catalyst less than 0.4 moles of said acid and/or lactone are passed over the catalyst.

8. Dehydrogenation catalyst suitable for application in the process according to any of the claims 1—7, characterized in that in the catalyst platinum, silicon dioxide and thorium oxide are present, and the catalyst is prepared by precipitating the platinum on the combination of said oxides.

9. Catalyst according to claim 8, characterized in that the catalyst contains 0.5—5% by weight of platinum and 5—15 grams of thorium oxide per 100 grams of silicon dioxide.

10. Catalyst according to any of the claims 8 or 9, characterized in that the catalyst contains silicon dioxide having an average pore radius between 2 and 5 nanometers.

## Patentansprüche

1. Verfahren zur Herstellung eines gegebenenfalls Alkylsubstituierten Resorcins durch Umwandlung eines Esters der gegebenenfalls Alkyl-substituierten 4-Oxopentaan-1-Carbonsäure im gasförmigen Zustand unter Verwendung eines Dehydrierkatalysators, welches Verfahren dadurch gekennzeichnet ist, dass eines oder mehrere Metalle aus der Gruppe Platin, Palladium, Ruthenium, Iridium und Rhodium auf einer Träger substanz als Dehydrierkatalysator verwendet werden, und zwar mit als Trägersubstanz eine Kombination eines oder mehrerer Oxyde aus der Gruppe Siliziumdioxyd, Aluminiumoxyd und Titanoxyd sowie einem oder mehreren Oxyden aus der Gruppe Thoriumoxyd und Lanthanidoxyde, wobei der Dehydrierkatalysator durch Ausfällen des Katalysators auf der Oxydkombination hergestellt wird.

2. Verfahren nach Anspruch 1, dadurch gekennzeichnet, dass ein Dehydrierkatalysator verwendet wird mit einem Gewichtsanteil von 0,5—5% eines Metalls der genannten Metallgruppe.

3. Verfahren nach Anspruch 1 oder 2, dadurch gekennzeichnet, dass Platin als Metall verwendet wird.

4. Verfahren nach einem der Ansprüche 1—3, dadurch gekennzeichnet, dass die Kombination Siliziumdioxyd-Thoriumoxyd als Trägersubstanz verwendet wird.

5. Verfahren nach Anspruch 4, dadurch gekennzeichnet, dass das Siliziumdioxyd einen mitleren Porendurchmesser zwischen 2 und 5 Nanometern hat.

6. Verfahren nach einem der Ansprüche 4 oder 5, dadurch gekennzeichnet, dass 5—15 Gramm Thoriumoxyd je 100 Gram Siliziumdioxyd verwendet werden.

7. Verfahren nach einem der Ansprüche 1—6, dadurch gekennzeichnet, dass die Säure und/oder das Lacton, die während der Umwandlung des Esters gebildet werden, rückgeführt und je Mol des Esters, der über den Katalysator geleitet wird, weniger als 0,4 Mol der genannten Säure und/oder des genannten Lactons über den Katalysator geleitet wird.

8. Dehydrierkatalysator, geeignet für die Anwendung im Verfahren nach einem der Ansprüche 1—7, dadurch gekennzeichnet, dass im Katalysator Platin, Siliziumdioxyd und Thoriumoxyd anwesend sind und dass der Katalysator durch Ausfällen des Platins auf der genannten Oxydkombination hergestellt wird.

9. Katalysator nach Anspruch 8, dadurch gekennzeichnet, dass der Katalysator 0,5—5 Gew.-% Platin und 5—15 Gramm Thoriumoxyd je 100 Gramm Siliziumdioxyd enthält.

10. Katalysator nach einem der Ansprüche 8 oder 9, dadurch gekennzeichnet, dass der Katalysator ein Siliziumdioxyd mit einem mittleren Porendurchmesser zwischen 2 und 5 Nanometern enthält.

# O 021 511

**Revendications**

1. Procédé de préparation de résorcinol éventuellement alcoylé par la conversion en phase gazeuse d'un ester d'acide 4-oxopentane-1-carboxylique éventuellement alcoylé en utilisant un catalyseur de déshydrogénation, caractérisé en ce qu'un ou plusieurs métaux, choisis dans le groupe composé de platine, palladium, ruthénium, iridium et rhodium sur un support, sont utilisés comme catalyseur de déshydrogénation, le support étant une combinaison d'un ou de plusieurs oxydes choisis dans le groupe composé de bioxyde de silicium, oxyde d'aluminium et oxyde de titane et d'un ou de plusieurs oxydes choisis dans le groupe composé d'oxyde de thorium et d'oxydes de lanthanide, alors que le catalyseur de déshydrogénation est préparé en faisant précipiter celui-ci sur la combinaison d'oxydes.

2. Procédé selon la revendication 1, caractérisé en ce qu'on utilise un catalyseur de déshydrogénation ayant une teneur de 0,5—5% en poids d'un métal dudit groupe de métaux.

3. Procédé selon l'une des revendications 1 à 2, caractérisé en ce qu'on utilise du platine comme métal.

4. Procédé selon l'une des revendications 1 à 3, caractérisé en ce qu'on utilise comme support, la combinaison de bioxyde de silicium et d'oxyde de thorium.

5. Procédé selon la revendication 4, caractérisé en ce qu'on utilise du bioxyde de silicium ayant un rayon moyen des pores situé entre 2 et 5 nanomètres.

6. Procédé selon l'une des revendications 4 à 5, caractérisé en ce qu'on utilise 5—15 g d'oxyde de thorium par 100 g de bioxyde de silicium.

7. Procédé selon l'une des revendications 1 à 6, caractérisé en ce que l'acide et/ou la lactone formés pendant la conversion de l'ester est mis en recirculation et que, par mole d'ester à conduire sur le catalyseur, on fait passer sur celui-ci moins de 0,4 mole dudit acide et/ou de ladite lactone.

8. Catalyseur de déshydrogénation qui convient à l'application dans le procédé selon l'une ou l'autre des revendications 1 à 7, caractérisé en ce que le catalyseur contient du platine, du bioxyde de silicium et de l'oxyde de thorium et que le catalyseur est préparé en faisant précipiter le platine sur la combinaison desdits oxydes.

9. Catalyseur selon la revendication 8, caractérisé en ce que le catalyseur contient 0,5—5% en poids de platine et 5—15 g d'oxyde de thorium par 100 g de bioxyde de silicium.

10. Catalyseur selon l'une ou l'autre des revendications 8 et 9, caractérisé en ce que le catalyseur contient du bioxyde de silicium ayant un rayon moyen des pores situé entre 2 et 5 nanomètres.